(19) Europäisches Patentamt
European Patent Office
Office européen des brevets

(11) **EP 2 118 648 B1**

(12) **FASCICULE DE BREVET EUROPEEN**

(45) Date de publication et mention
de la délivrance du brevet:
**16.06.2010 Bulletin 2010/24**

(21) Numéro de dépôt: 08761976.3

(22) Date de dépôt: **24.01.2008**

(51) Int Cl.:
***G01N 33/18*** *(2006.01)*     ***G01N 21/33*** *(2006.01)*
***C02F 1/00*** *(2006.01)*

(86) Numéro de dépôt international:
**PCT/FR2008/050110**

(87) Numéro de publication internationale:
**WO 2008/104647 (04.09.2008 Gazette 2008/36)**

(54) **PROCEDE DE QUALIFICATION DE LA VARIABILITE DE LA COMPOSITION D'UN EFFLUENT**

VERFAHREN ZUR BEURTEILUNG DER VARIABILITÄT EINER ABWASSERZUSAMMENSETZUNG

METHOD FOR QUALIFYING THE VARIABILITY OF AN EFFLUENT COMPOSITION

(84) Etats contractants désignés:
**AT BE BG CH CY CZ DE DK EE ES FI FR GB GR
HR HU IE IS IT LI LT LU LV MC MT NL NO PL PT
RO SE SI SK TR**

(30) Priorité: **25.01.2007  FR 0752882**

(43) Date de publication de la demande:
**18.11.2009  Bulletin 2009/47**

(73) Titulaire: **VEOLIA EAU - COMPAGNIE GENERALE
DES EAUX
75008 Paris (FR)**

(72) Inventeurs:
• **DENIEUL, Marie-Pierre**
  **F-92110 Clichy (FR)**
• **DANIEL, Olivier**
  **F-77185 Lognes (FR)**
• **BUCAILLE, Arnaud**
  **Causeway Bay, Hong Kong (HK)**
• **LEMOINE, Cyrille**
  **F-78500 Sartrouville (FR)**

(74) Mandataire: **Balesta, Pierre et al
Cabinet Beau de Loménie
158, rue de l'Université
75340 Paris Cedex 07 (FR)**

(56) Documents cités:
EP-A1- 0 707 247     WO-A-98/40721
DE-A1- 19 645 246    FR-A1- 2 783 322
US-A- 5 870 692      US-A1- 2003 236 649
US-B1- 6 351 986

EP 2 118 648 B1

**Description**

La présente invention concerne le domaine de l'analyse et du contrôle en ligne de la composition des effluents liquides ou gazeux, telles par exemple l'eau potable, les eaux urbaines ou industrielles.

La présente invention vise plus particulièrement un procédé de détection de la variabilité de la composition d'un tel effluent qu'il soit liquide ou gazeux.

Dans les procédés de détection déjà connus, les mesures sont effectuées en ligne, par exemple dans une conduite disposée en amont d'une station de traitement d'eau usée.

Un tel procédé permet de détecter une anormalité dans la composition de l'effluent, notamment physico-chimique ou biologique, susceptible de provoquer un incident dans le procédé de traitement situé en aval du lieu où sont effectuées les mesures.

L'acquisition en ligne de ces mesures et leur traitement mathématique permettent d'alerter l'opérateur de l'arrivée d'un effluent ayant une composition différente de celle de l'effluent alimentant habituellement la station de traitement. Le cas échéant, l'opérateur prendra des mesures appropriées, par exemple en déviant l'effluent « différent » vers une autre unité de traitement et/ou en prélevant un échantillon de cet effluent qui sera analysé a posteriori.

Parmi les procédés de détection de la variabilité en ligne d'un effluent, certains mettent en oeuvre la spectrophotométrie Ultra-Violet visible (ci-après dénommée « UV-VIS ») permettant à partir d'un ensemble de signaux, de représenter la variabilité qualitative de l'effluent.

Pour les aspects qualitatifs, le spectre UV-VIS fait office « d'empreinte digitale » de l'effluent.

En d'autres termes, un effluent donné possède un spectre UV-VIS propre comprenant des points particuliers (dénommés points isobestiques) qui, en toute théorie, ne varient pas au cours du temps.

Dès lors que l'on détecte un changement dans l'allure du spectre UV mesuré en ligne, on comprend que ce changement d'allure traduit une modification de la composition de l'effluent.

L'information obtenue indique à l'opérateur s'il y a conservation globale ou non de la qualité de l'effluent mais ne permet pas d'évaluer la dangerosité de l'effluent en question. Enfin, ces points n'existent pas forcément pour tous les effluents, ce qui rend inefficace cette méthode dans ce cas.

Un autre mode connu de traitement des spectres UV, tel que décrit dans les documents FR 2 783 322 et FR 2 787 883, consiste à utiliser la déconvolution des spectres UV, ce traitement se basant sur l'hypothèse selon laquelle chaque spectre UV d'un effluent peut être considéré comme une combinaison linéaire d'un petit nombre de spectres UV connus, appelés spectres de référence, et notamment relatifs à des composés particuliers de l'effluent étudié.

Cette méthode suppose donc d'avoir une connaissance a priori de la composition de l'effluent ou des composés susceptibles de s'y trouver, ce qui impose de réaliser des analyses en laboratoires préalables. En outre, une bibliothèque de spectres de composés ou de compositions prédéterminés doit être préalablement réalisée.

Par ailleurs, le document EP 1 070 954 mentionne l'utilisation de la spectroscopie dans le spectre « UV-visible » pour la détermination par déconvolution de spectres de paramètres globaux. Ce document propose une surveillance temporelle de l'effluent de manière à déterminer le moment précis où une pollution éventuelle commence à se manifester. Pour chacun des sites sur lequel on réalisera les mesures, cette méthode nécessite donc une caractérisation préalable de l'effluent via des méthodes conventionnelles, ainsi que la calibration et l'identification préalable de spectres de référence spécifiques à l'effluent analysé.

Il en résulte que les procédés de qualification connus sont totalement liés à l'environnement dans lequel ils sont destinés à être mis en oeuvre. En particulier, dans le cas de la spectrophotométrie UV-VIS, cela suppose que l'effluent absorbe dans la gamme de longueur d'onde considérée.

Un autre inconvénient des procédés de l'art antérieur est qu'ils ne sont pas capables de traiter d'autres types de mesures, tels notamment le pH, la conductivité, ou la température.

Un but de la présente invention est de proposer un procédé de qualification de la variabilité de la composition d'un effluent, dans lequel on réalise une succession de mesures au cours du temps d'au moins un premier et un deuxième paramètres de cet effluent, lequel procédé est indépendant de l'environnement dans lequel il est mis en oeuvre tout en étant capable de traiter des mesures tant paramétriques que non-paramétriques.

L'invention atteint son but par le fait que, à chaque pas de temps :

- on détermine une première dérivée pour chacun des premier et deuxième paramètres ;
- on détermine au moins une deuxième dérivée entre les premier et deuxième paramètres ;
- on définit, pour la première dérivée de chacun des paramètres, des premiers domaines logiques comprenant au moins un premier domaine logique normal correspondant à une variabilité normale de ladite première dérivée, et un premier domaine logique anormal correspondant à une variabilité anormale de ladite première dérivée ;
- on attribue des premières probabilités d'appartenance à chacun des premiers domaines logiques pour la première dérivée de chacun des paramètres ;
- on définit des deuxièmes domaines logiques comprenant au moins un deuxième domaine logique normal correspondant à une variabilité normale de ladite deuxième dérivée, et un deuxième domaine logique anormal correspondant à une variabilité anormale de ladite deuxième dérivée ;

- on attribue des deuxièmes probabilités d'appartenance de la deuxième dérivée à chacun des deuxièmes domaines logiques ;
- on définit des domaines logiques globaux à partir des premiers et deuxièmes domaines logiques, les domaines logiques globaux comprenant au moins un domaine logique global normal et un domaine logique global anormal ;
- on attribue des probabilités d'appartenance globales de l'ensemble constitué par les premières dérivées des paramètres et au moins une deuxième dérivée à chacun des domaines logiques globaux ;
- on qualifie la variabilité de la composition de l'effluent à partir de ces probabilités d'appartenance globales.

Ainsi, grâce à l'invention, il n'est pas nécessaire de connaître a priori la composition « habituelle » de l'effluent pour pouvoir détecter une anomalie dans la variabilité de sa composition.

En outre, grâce à l'invention, il n'est pas non plus nécessaire de procéder à des analyses préalables afin d'initialiser le procédé.

Tout au contraire, le procédé selon l'invention ne nécessite qu'une succession temporelle de mesures de paramètres pour s'adapter à l'environnement dans lequel il est mis en oeuvre, ledit environnement pouvant être par exemple un réseau d'assainissement ou un réseau d'eau potable ou bien encore un site industriel.

Cela signifie que le procédé selon la présente invention peut être mis en oeuvre de façon simple et rapide, sans nécessiter les étapes préalables de calibration ou d'analyse en laboratoire telles que celles effectuées dans les procédés connus.

Par ailleurs, le procédé selon l'invention peut avantageusement se baser tant sur l'analyse de la variabilité de mesures de spectres, que sur l'analyse de la variabilité de mesures de grandeurs, tels par exemple mais non exclusivement le pH, le potentiel Rédox, la conductivité ou la température.

Un des principaux objectifs de l'invention est de renseigner immédiatement et en temps réel l'opérateur de l'arrivée éventuelle d'un effluent ayant une composition à risque.

Pour ce faire, on analyse l'évolution des variations de chacun des paramètres pris séparément d'une part, ainsi que l'évolution de la variation relative entre les premier et deuxième paramètres d'autre part.

Au sens de la présente invention, les premières dérivées sont prises par rapport à une même grandeur, de préférence le temps, tandis que les deuxièmes dérivées dénotent la variation d'un des paramètres par rapport à l'autre, de préférence au cours du temps.

Dans le cas particulier où le paramètre dépend d'une variable non temporelle, la deuxième dérivée, pourra être par exemple la dérivée du paramètre par rapport à ladite variable, pour un pas de temps donné.

Autrement dit, on analyse d'une part, à l'aide des premières dérivées, comment chacun des paramètres évolue, de préférence dans le temps. On parlera ici de variances individuelles des paramètres, la première dérivée étant avantageusement une dérivée temporelle.

D'autre part, on analyse comment les paramètres évoluent l'un par rapport à l'autre à l'aide des deuxièmes dérivées. On parlera ici de variances croisées desdits paramètres.

Au demeurant, les variances croisées permettent notamment de déterminer si les évolutions des paramètres sont similaires ou à tout le moins corrélées.

De manière préférentielle, le nombre de paramètres est sensiblement plus grand que deux.

De manière préférentielle, il existe autant de deuxièmes dérivées que de combinaisons entre les paramètres.

L'idée sous jacente est de comparer les variances individuelles et croisées de manière à établir une indication de la variance globale de la composition de l'effluent.

Le traitement mathématique permettant la mise en oeuvre du procédé selon la présente invention repose sur la théorie de la logique floue, bien connue par ailleurs, si bien que la comparaison mentionnée ci-dessus sera obtenue par une opération d'inférence, bien connue par ailleurs.

En fonction de l'indication de variance globale, on pourra qualifier la variation de la composition de l'effluent de normal ou d'anormal, ou par tout autre qualificatif approprié.

De manière plus précise, l'attribution des premières probabilités d'appartenance à chacun des premiers domaines logiques pour la première dérivée de chacun des paramètres permet de caractériser la variance individuelle des paramètres, tandis que l'attribution des deuxièmes probabilités d'appartenance de la deuxième dérivée à chacun des deuxièmes domaines logiques permet quant à elle de caractériser la variance croisée des paramètres.

Enfin, on comprend que l'étape d'attribution des probabilités d'appartenance globales permet de caractériser la variance globale des paramètres.

Le traitement mathématique appliqué dans la présente invention utilise préférentiellement au moins une base de données glissante dont la longueur est variable, la base de données étant constituée par les valeurs des paramètres à des pas de temps précédents, grâce à quoi le procédé s'adapte seul aux évolutions naturelles de l'effluent.

Par « glissante », on entend que la base de données contient un nombre prédéterminé de valeurs, une nouvelle entrée dans la base s'accompagnant de la suppression de la plus ancienne valeur stockée.

De manière particulièrement intéressante, cela implique que l'opérateur n'a pas à fixer de seuils pour déterminer si l'effluent varie, ni d'adapter un tel seuil à l'évolution naturelle de l'effluent.

Au contraire, grâce à l'invention, de tels seuils sont déterminés automatiquement par le procédé, en fonction des données enregistrées dans la base de données.

Comme on l'a déjà mentionné, le procédé selon l'invention ne nécessite aucune mesure, préalable ou non, de l'effluent en laboratoire. Par ailleurs, aucune calibration préalable de l'outil mathématique utilisé n'est nécessaire.

Ainsi s'affranchit-on de mesures ciblées sur certaines caractéristiques de l'effluent, tant et si bien que le procédé selon l'invention offre une approche globale du risque de dangerosité lié à la variabilité de la composition de l'effluent.

Enfin, on pourra choisir un critère de qualification simple, par exemple un « signal rouge » signifiant que l'effluent a fortement varié, ceci impliquant un risque fort de dysfonctionnement du traitement, associé par exemple à un « signal vert » signifiant que l'effluent n'a pas ou peu varié.

Avantageusement, pour la première dérivée de chacun des paramètres, les premiers domaines logiques sont définis à partir d'un multiple de l'écart-type évalué à partir d'un ensemble constitué par des valeurs de la première dérivée dudit paramètre déterminées à des pas de temps précédents.

De manière préférentielle, pour la première dérivée de chacun des paramètres, les premiers domaines logiques sont également définis à partir de la moyenne ou de la médiane d'un ensemble constitué par des valeurs de la première dérivée dudit paramètre déterminées à des pas de temps précédents.

Avantageusement, les deuxièmes domaines logiques sont définis à partir d'un multiple de l'écart type évalué à partir d'un ensemble constitué par des valeurs de ladite au moins une deuxième dérivée déterminées à des pas de temps précédents.

Avantageusement, les domaines logiques globaux sont définis à partir d'un multiple de l'écart-type évalué à partir d'un ensemble constitué par les valeurs des premières dérivées des paramètres et des valeurs de ladite au moins une deuxième dérivée déterminées à des pas de temps précédents.

Les valeurs déterminées à des pas de temps précédents mentionnées ci-dessus sont avantageusement stockées dans au moins une base de données glissante, telle que celle mentionnée ci-dessus.

En vue d'affiner la qualification de la variabilité de la composition de l'effluent, on définit en outre, pour la première dérivée de chacun des paramètres, un premier domaine logique intermédiaire correspondant à une variabilité peu normale de ladite première dérivée.

On définit en outre, pour ladite au moins une deuxième dérivée, un deuxième domaine logique intermédiaire correspondant à une variabilité peu normale de ladite deuxième dérivée.

De surcroît, on peut également définir un ou plusieurs domaines logiques globaux intermédiaires, correspondant à une variation peu normale de la composition de l'effluent.

En pratique, on pourra utiliser un « signal orange » complétant les signaux rouge et vert mentionnés ci-dessus, ledit signal orange traduisant une variation de l'effluent dans un domaine acceptable, le risque étant estimé comme étant modéré mais nécessitant une vigilance accrue (risque potentiel d'évolution vers un signal rouge).

Selon un premier mode de réalisation, les probabilités d'appartenance globales sont calculées à partir d'une somme pondérée des premières et deuxièmes probabilités d'appartenance.

Selon un second mode de réalisation, on définit en outre :

- des troisièmes domaines logiques comprenant au moins un troisième domaine logique normal et un troisième domaine logique anormal, les troisièmes domaines logiques étant définis à partir des premiers domaines logiques ;
- des quatrièmes domaines logiques comprenant au moins un quatrième domaine logique normal et un quatrième domaine logique anormal, les quatrièmes domaines logiques étant définis à partir des deuxièmes domaines logiques ;
- on attribue en outre des troisièmes probabilités d'appartenance d'un ensemble constitué par les premières dérivées des paramètres à chacun des troisièmes domaines logiques ;
- on attribue en outre des quatrièmes probabilités d'appartenance d'un ensemble constitué par au moins ladite au moins une deuxième dérivée à chacun des quatrièmes domaines logiques ;
- les domaines logiques globaux étant définis à partir des troisièmes et quatrièmes domaines logiques.

Dans ce second mode de réalisation, on insère des domaines logiques supplémentaires, à savoir les troisièmes et quatrièmes domaines logiques de manière à affiner la qualification de la variabilité de la composition de l'effluent et à éviter les fausses alertes.

De manière préférentielle, le troisième domaine logique « normal » correspond à l'union des premiers domaines logiques « normaux », le troisième domaine logique « anormal » correspond à l'union des premiers domaines logiques « anormaux », le quatrième domaine logique « normal » correspond à l'union des deuxièmes domaines logiques « normaux », tandis que le quatrième domaine logique « anormal » correspond à l'union des deuxièmes domaines logiques « anormaux ».

Avantageusement, on définit en outre un troisième domaine logique « peu normal » qui correspond de préférence à l'union des premiers domaines logiques « peu normaux », et un quatrième domaine logique « peu normal » qui correspond de préférence à l'union des deuxièmes domaines logiques « peu normaux ».

De manière préférentielle, on définit cinq domaines logiques globaux.

Le premier domaine logique global correspond de préférence à l'union du troisième domaine logique « normal » et du quatrième domaine logique « normal ».

Le deuxième domaine logique global correspond de préférence à l'union de l'union du troisième domaine logique « normal » et du quatrième domaine logique « peu normal » et de l'union du troisième domaine logique « peu normal » et du quatrième domaine logique « normal ».

Le troisième domaine logique global correspond de préférence à l'union de l'union du troisième domaine logique « normal » et du quatrième domaine logique « anormal » avec l'union du troisième domaine logique « peu normal » et du quatrième domaine logique « peu normal », et de l'union du troisième domaine logique « anormal » et du quatrième domaine logique « normal ».

Le quatrième domaine logique global correspond de préférence à l'union de l'union du troisième domaine logique « peu normal » et du quatrième domaine logique « anormal », et de l'union du troisième domaine logique « anormal » et du quatrième domaine logique « peu normal ».

Le cinquième domaine logique global correspond de préférence à l'union du troisième domaine logique « anormal » et du quatrième domaine logique « anormal ».

Enfin, après les avoir calculées, on attribue les probabilités d'appartenance globales de l'ensemble constitué par les premières dérivées des paramètres et les secondes dérivées aux domaines logiques globaux.

Avantageusement, les probabilités d'appartenance globales sont calculées à partir d'une somme pondérée des troisièmes et quatrièmes probabilités d'appartenance.

De manière préférentielle, ladite somme pondérée fait intervenir au moins un coefficient de pondération dynamique, par exemple l'écart-type évalué à partir d'un ensemble constitué par des valeurs de la première dérivée déterminées à des pas de temps précédents.

Selon une première variante, le domaine logique global qualifiant la variabilité de la composition de l'effluent est celui pour lequel la probabilité d'appartenance globale est maximale.

De manière préférentielle, seul le cinquième domaine logique global est utilisé pour déterminer la variation de la composition de l'effluent.

On comprend que ce cinquième domaine logique global constitue celui pour lequel la probabilité d'une variabilité particulièrement anormale de la composition de l'effluent est la plus forte.

Selon une deuxième variante, le domaine logique global qualifiant la variabilité de la composition de l'effluent est le domaine logique le plus « anormal » pour lequel la probabilité d'appartenance globale est non nulle.

Selon une troisième variante, le domaine logique global qualifiant la variabilité de la composition de l'effluent est le domaine logique le plus anormal pour lequel la probabilité d'appartenance globale est supérieure à un seuil prédéterminé.

Selon un mode de réalisation préférentiel de l'invention, le premier paramètre est l'absorbance de l'effluent mesuré pour une première longueur d'onde.

De manière préférentielle, chacun des paramètres correspond à l'absorbance de l'effluent pour une longueur d'onde donnée.

Préférentiellement, on mesure à chaque pas de temps l'absorbance de l'effluent pour une pluralité de longueurs d'onde comprises de préférence entre 200 nm et 700 nm.

Avantageusement, la première dérivée est une dérivée de l'absorbance par rapport au temps pour une longueur d'onde donnée, tandis que la deuxième dérivée est une dérivée de l'absorbance par rapport à la longueur d'onde calculée à un pas de temps donné.

La présente invention concerne également un dispositif de suivi de la variabilité de la composition d'un effluent mettant en oeuvre le procédé selon la présente invention.

D'autres caractéristiques et avantages de l'invention apparaîtront mieux à la lecture de la description qui suit d'un mode de réalisation de l'invention donné à titre d'exemple non limitatif.

La description se réfère aux figures annexées sur lesquelles :

- la figure 1 est une représentation schématique d'un dispositif de suivi de la variabilité de la composition d'un effluent mettant en oeuvre le procédé selon la présente invention ;
- la figure 2 est un diagramme représentant les étapes du mode de mise en oeuvre préféré de la présente invention ;
- la figure 3 est un graphique représentant des fonctions d'appartenance en triangle permettant de déterminer des probabilités d'appartenance des premières dérivées aux premiers domaines logiques ; et
- la figure 4 est un graphique représentant des fonctions d'appartenance en triangle permettant de déterminer des probabilités d'appartenance des deuxièmes dérivées aux deuxièmes domaines logiques.

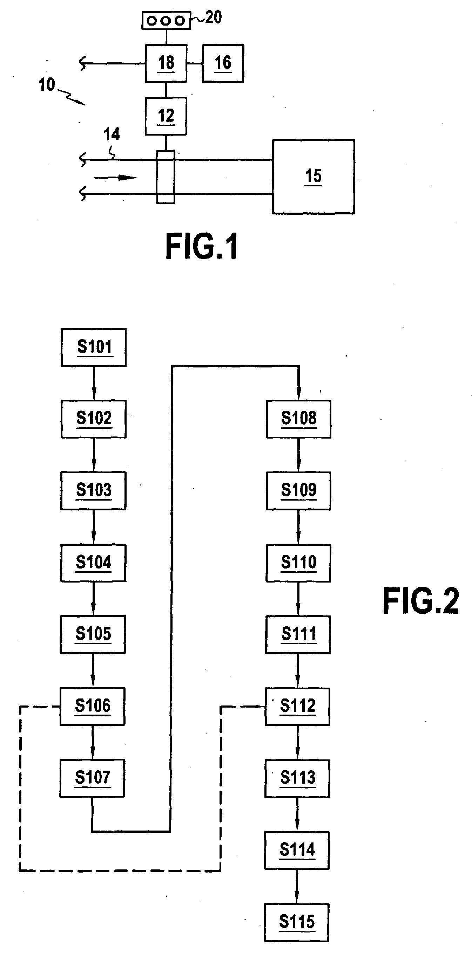

A l'aide des figures 1 à 4, on va maintenant décrire plus en détail un mode préféré de mise en oeuvre du procédé selon la présente invention dont l'objectif est d'évaluer la variabilité temporelle de la composition d'un effluent de l'industrie chimique.

La figure 1 représente un dispositif **10** conforme à l'invention mettant en oeuvre le procédé selon l'invention.

Comme on le voit sur cette figure, le dispositif **10** comprend des moyens de mesure **12** destinés à mesurer l'absorbance de l'effluent s'écoulant dans une conduite **14,** cette dernière étant préférentiellement mais non exclusivement disposée en amont d'une station de traitement **15** de l'effluent.

Les valeurs mesurées par les moyens de mesure **12** sont stockées dans des moyens de mémoire **16,** tandis qu'un microprocesseur **18** est prévu pour effectuer des opérations de calcul conduisant à la qualification de la variabilité de la composition de l'effluent.

Le résultat des opérations de calcul est préférentiellement donné par une interface visuelle **20** comportant trois feux de couleur, par exemple vert, orange et rouge caractérisant le niveau de normalité de la variabilité de l'effluent.

Le procédé selon l'invention va maintenant être expliqué plus en détail à l'aide du diagramme représenté sur la figure 2.

Dans ce mode de mise en oeuvre, on réalise au cours du temps une succession de mesures d'une pluralité de paramètres, à savoir l'absorbance de l'effluent pour plusieurs longueurs d'onde.

Autrement dit, à chaque pas de temps, on mesure, au cours d'une étape **S101,** l'absorbance de l'effluent pour plusieurs longueurs d'onde, celles-ci étant comprises entre 200 et 700 nm, de préférence entre 230 et 500 nm.

De préférence, les mesures sont effectuées en faisant varier la longueur d'onde par pas de 1 nm; tandis qu'un pas de temps $t$ dure deux heures.

Le diagramme de la figure 2 représente les étapes qui sont réalisées à chaque pas de temps.

Ainsi dispose-t-on, dans cet exemple de mise en oeuvre où l'on mesure l'absorbance entre 230 nm et 500 nm, de 271 paramètres qui ont été mesurés simultanément toutes les deux heures sur l'effluent. On comprend donc qu'au sens de l'invention, le premier paramètre est l'absorbance à la longueur d'onde de 230 nm, le deuxième paramètre est l'absorbance à la longueur d'onde de 231 nm, ainsi de suite jusqu'au 271ème paramètre, qui est l'absorbance à 500 nm.

Evidemment, le nombre de paramètres n'est pas limité à 271 et l'on peut tout à fait choisir un autre nombre de paramètres sans sortir du cadre de la présente invention.

De manière préférentielle mais non nécessairement, les moyens de mémoire comportent une base de données stockant les mesures de paramètres pour une pluralité de pas de temps $t.$

Au cours d'une seconde étape **S102,** on détermine une première dérivée pour chacun des 271 paramètres en calculant la différence entre deux valeurs d'absorbance consécutives dans le temps à une même longueur d'onde et en divisant le résultat obtenu par un écart de temps $\Delta t$ existant entre deux mesures.

Ainsi, si l'on considère le premier paramètre, à savoir l'absorbance $Abs$ de l'effluent à 230 nm, la première dérivée du 1er paramètre $d_{1,1}(t)$ a pour expression :

$$d_{1,1}(t) = \frac{Abs_{230}(t) - Abs_{230}(t - \Delta t)}{\Delta t}$$

Il en est de même pour la première dérivée du 2ème paramètre $d_{1,2}(t)$, qui a pour expression :

$$d_{1,2}(t) = \frac{Abs_{231}(t) - Abs_{231}(t - \Delta t)}{\Delta t}$$

Et ainsi de suite jusqu'au 271ème paramètre.

Au cours d'une troisième étape **S103,** on détermine au moins une deuxième dérivée entre les paramètres.

Plus précisément, on définit, dans cet exemple, 270 deuxièmes dérivées des 271 paramètres. La 1ère deuxième dérivée $d_{2,1}(t)$ est calculée comme étant la différence entre les valeurs d'absorbance à 231 nm et à 230 nm, soit :

$$d_{2,1}(t) = \frac{Abs_{231}(t) - Abs_{230}(t)}{231 - 230}$$

On comprend donc que la deuxième dérivée d$_{2,1}$(t) dénote bien l'écart relatif entre les paramètres Abs$_{231}$(t) et Abs$_{230}$(t) et que la fonction t-> d$_{2,1}$(t) fournit la variation temporelle de l'écart relatif entre ces deux paramètres.

Il en est de même pour la 2$^{nde}$ deuxième dérivée d$_{2,2}$(t), qui a pour expression :

$$d_{2,2}(t) = \frac{Abs_{232}(t) - Abs_{231}(t)}{232 - 231}$$

Et ainsi de suite jusqu'à la 270$^{ème}$ deuxième dérivée.

Dans une quatrième étape **S104,** on définit pour chaque j$^{ème}$ première dérivée (j=1 à 271), c'est-à-dire pour la première dérivée de chacun des paramètres, trois premiers domaines logiques ($DL_1^i(j)$, i=1 à 3, j=1 à 271) déterminés à partir des calculs suivants :

a) on calcule une moyenne $\overline{d_{1,j}}$(t) et un écart-type $\sigma_{d_{1,j}}$(t) à partir des 50 valeurs calculées antérieurement de chaque j$^{ème}$ première dérivée (j = 1 à 271) :

$$\overline{d_{1,j}}(t) = \frac{1}{50}\sum_{i=0}^{49} d_{1,j}(t - i\Delta t)$$

et :

$$\sigma_{d_{1,j}}(t) = \sqrt{\frac{1}{49}\sum_{i=0}^{49}\left(d_{1,j}(t - i\Delta t) - \overline{d_{1,j}}(t)\right)^2}$$

b) on calcule pour chaque j$^{ème}$ première dérivée (j = 1 à 271) l'écart absolu $\varepsilon_{1,j}$(t) à la moyenne des premières dérivées :

$$\varepsilon_{1,j}(t) = \left|d_{1,j}(t) - \overline{d_{1,j}}(t)\right|$$

c) Les premiers domaines logiques $DL_1^i(j,t)$ (avec i=1 à 3, j=1 à 271) sont alors définis au pas de temps *t* comme suit :

- le 1$^{er}$ des premiers domaines logiques de chaque j$^{ème}$ première dérivée « variation normale de la première dérivé », $DL_1^1(j,t)$, est **caractérisé en ce que** : $\varepsilon_{1,j}$(t) < $\sigma_{1,j}$(t) ;
- le 2$^{ème}$ des premiers domaines logiques de chaque j$^{ème}$ première dérivée « variation peu normale de la première dérivée », $DL_1^2(j,t)$, est **caractérisé en ce que** : $\sigma_{1,j}$(t) < $\varepsilon_{1,k}$(t) < 2$\sigma_{1,j}$(t);

- le 3ème des premiers domaines logiques de chaque jème première dérivée « variation anormale de la première dérivée », $DL_1^3(j,t)$, est **caractérisé en ce que** : $\varepsilon_{1,j}(t) > 2\sigma_{1,j}(t)$.

Au cours d'une cinquième étape **S105,** on attribue des probabilités d'appartenance à l'instant t des 271 premières dérivées à chacun des premiers domaines logiques, $P\left(DL_1^i(j,t)\right)$ (i=1 à 3, j=1 à 271), en effectuant les sous-étapes suivantes :

a) on norme entre -1 et 1 l'écart absolu obtenu pour chaque jème première dérivée, avec la correspondance suivante :

- Si $\varepsilon_{1,j}(t) = 0$ alors $\|\varepsilon_{1,j}(t)\| = -1$;
- Si $\varepsilon_{1,j}(t) \geq 3\sigma_{1,j}(t).d_{1,j}(t)$ alors $\|\varepsilon_{1,j}(t)\| = +1$;

- Sinon $\left\|\varepsilon_{1,j}(t)\right\| = -1 + \dfrac{2 \times \varepsilon_{1,j}(t)}{3\sigma_{1,j}(t)}$

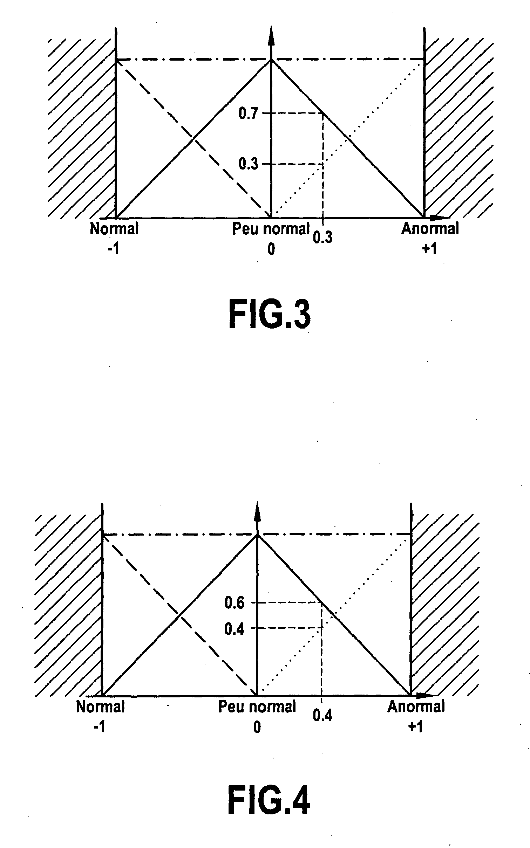

b) on procède à la fuzzyfication (*i.e.* à l'attribution des probabilités d'appartenance de chaque jème première dérivée aux premiers domaines logiques $DL_1^i(j,t)$ de chaque valeur normée $\|\varepsilon_{1,j}(t)\|$ avec des fonctions d'appartenance en triangle telles que représentées sur la figure 3.

Sur la figure 3, on a représenté le cas où, pour la 50ème première dérivée avec une valeur de $\|\varepsilon_{1,50}(t_0)\| = 0.3$ à l'instant $t_0$, on obtient $P\left(DL_1^1(50,t_0)\right) = 0$, $P\left(DL_1^2(50,t_0)\right) = 0.7$, $P\left(DL_1^3(50,t_0)\right) = 0.3$, qui correspondent aux probabilités d'appartenance de la 50ème première dérivée aux premiers domaines logiques.

Au cours d'une sixième étape **S106,** on définit des deuxièmes domaines logiques, à savoir, pour chaque kème deuxième dérivée trois deuxièmes domaines logiques ( $DL_2^i(k,t)$, i=1 à 3, k=1 à 270), déterminés à partir des calculs suivants :

a) on calcule la moyenne et l'écart-type des 50 dernières valeurs de chaque deuxième dérivée :

$$\overline{d_{2,k}}(t) = \frac{1}{50}\sum_{i=0}^{49} d_{2,k}(t - i\Delta t)$$

et :

$$\sigma_{d_{2,k}}(t) = \sqrt{\frac{1}{49}\sum_{i=0}^{49}\left(d_{2,k}(t - i\Delta t) - \overline{d_{2,k}}(t)\right)^2}$$

b) on calcule pour chaque kème deuxième dérivée l'écart absolu $\varepsilon_{2,k}(t)$ à la moyenne des deuxièmes dérivées :

$$\varepsilon_{2,k}(t) = \left|d_{2,k}(t) - \overline{d_{2,k}}(t)\right|.$$

c) les deuxièmes domaines logiques $DL_2^i(k,t)$ sont alors définis comme suit :

- le 1$^{er}$ des deuxièmes domaines logiques « variation normale de la k$^{ème}$ deuxième dérivée », $DL_2^1(k,t)$, est **caractérisé en ce que** $\varepsilon_{2,k}(t) < \sigma_{2,k}(t)$ ;

- le 2$^{ème}$ des deuxièmes domaines logiques « variation peu normale de la k$^{ème}$ deuxième dérivée », $DL_2^2(k,t)$, est **caractérisé en ce que** $\sigma_{2,k}(t) < \varepsilon_{2,k}(t) < 2\sigma_{2,k}(t)$;

- le 3$^{ème}$ des deuxièmes domaines logiques « variation anormale de la k$^{ème}$ deuxième dérivée », $DL_2^3(k,t)$, est caractérisé en ce que $\varepsilon_{2,k}(t) > 2\sigma_{2,k}(t)$.

Au cours d'une septième étape **S107,** on attribue les probabilités d'appartenance des k$^{ème}$ deuxièmes dérivées aux deuxièmes domaines logiques, $P\!\left(DL_2^i(k,t)\right)$ (i=1 à 3, k=1 à 270), en effectuant les sous-étapes suivantes :

a) on norme entre -1 et 1 l'écart absolu obtenu, avec la correspondance suivante :

- Si $\varepsilon_{2,k}(t) = 0$ alors $\|\varepsilon_{2,k}(t)\| = -1$;
- Si $\varepsilon_{2,k}(t) \geq 3\sigma_{2,k}(t).d_{2,k}(t)$ alors $\|\varepsilon_{2,k}(t)\| = +1$;

- Sinon $\left\|\varepsilon_{2,k}(t)\right\| = -1 + \dfrac{2 \times \varepsilon_{2,k}(t)}{3\sigma_{2,k}(t)}$

b) on procède à la fuzzyfication (*i.e.* à l'attribution des probabilités d'appartenance de chaque k$^{ème}$ deuxièmes dérivées aux deuxièmes domaines logiques $DL_2^i(k,t)$ de chaque valeur normée $\|\varepsilon_{2,k}(t)\|$ avec des fonctions d'appartenance en triangle telles que représentées sur la figure 4.
Sur la figure 4, on a représenté le cas où, pour la 50$^{ème}$ deuxième dérivée avec une valeur de $\|\varepsilon_{2,50}(t_0)\| = 0.4$ à l'instant $t_0$, on obtient $P\!\left(DL_2^1(50,t_0)\right) = 0$ , $P\!\left(DL_2^2(50,t_0)\right) = 0.6$ , $P\!\left(DL_2^3(50,t_0)\right) = 0.4$ , qui correspondent aux probabilités d'appartenance de la 50$^{ème}$ deuxième dérivée aux deuxièmes domaines logiques.

Au cours d'une huitième étape **S108,** préférentielle mais non nécessaire, on définit des troisièmes domaines logiques à savoir, trois troisièmes domaines logiques ( $DL_3^i(t)$ , i=1 à 3) pour les premières dérivées, ceux-ci correspondant à :

a) Pour le 1$^{er}$ des troisièmes domaines logiques $DL_3^1(t)$ à l'union des 1$^{ers}$ premiers domaines logiques « normaux » ;

b) Pour le 2$^{ème}$ des troisièmes domaines logiques $DL_3^2(t)$, à l'union des 2$^{èmes}$ premiers domaines logiques « peu normaux » ;

c) Pour le 3$^{ème}$ des troisièmes domaines logiques $DL_3^3(t)$, à l'union des 3$^{èmes}$ premiers domaines logiques « anormaux » ;

Au cours d'une neuvième étape **S109,** également préférentielle mais non nécessaire, on calcule la probabilité d'appartenance aux troisièmes domaines logiques de l'ensemble des 271 premières dérivées en faisant la somme des probabilités d'appartenance de chaque j$^{ième}$ première dérivée aux premiers domaines logiques pondérée par l'écart-type des premières dérivées :

$$P\!\left(DL_3^i(t)\right) = \sum_{j=1}^{271} \sigma_{1,j}(t) \times P\!\left(DL_1^i(j,t)\right),$$

avec i=1 à 3

Au cours d'une dixième étape **S110,** également préférentielle mais non nécessaire, on définit des quatrièmes domaines logiques, à savoir trois quatrièmes domaines logiques ( $DL_4^i(t)$, i=1 à 3) pour les deuxièmes dérivées, ceux-ci correspondant à :

a) Pour le 1er des quatrièmes domaines logiques, $DL_4^1(t)$, à l'union des 1ers deuxièmes domaines logiques « normaux » ;

b) Pour le 2ème des quatrièmes domaines logiques, $DL_4^2(t)$, à l'union des 2èmes deuxièmes domaines logiques « peu normaux » ;

c) Pour le 3ème des quatrièmes domaines logiques, $DL_4^3(t)$, à l'union des 3èmes deuxièmes domaines logiques « anormaux » ;

Au cours d'une onzième étape **S111,** également préférentielle mais non nécessaire, on calcule la probabilité d'appartenance de chaque 270 deuxièmes dérivées aux trois quatrièmes domaines logiques en faisant la somme des probabilité d'appartenance de chaque $k^{ème}$ deuxièmes dérivées aux deuxièmes domaines logiques pondérée par l'écart-type des deuxièmes dérivées :

$$P\left(DL_4^i(t)\right) = \sum_{k=1}^{270} \sigma_{2,k}(t) \times P\left(DL_2^i(k,t)\right),$$

avec i=1 à 3

Au cours d'une douzième étape **S112,** on définit des domaines logiques globaux à partir desquels on définira la variabilité globale de l'effluent, de préférence cinq domaines logiques globaux ( $DL_5^I(t)$, avec I=1 à 5) à partir des troisièmes et quatrièmes domaines logiques. Ils correspondent à :

a) Pour le 1er des domaines logiques globaux, $DL_5^I(t)$, à l'union du 1er troisième domaine logique « normal » et du 1er quatrième domaine logique « normal » ;

b) Pour le 2ème des domaines logiques globaux, $DL_5^2(t)$, à l'union de l'union du 1er troisième domaine logique « normal » et du 2ème quatrième domaine logique « peu normal » et de l'union du 2ème troisième domaine logique « peu normal » et du 1er quatrième domaine logique « normal » ;

c) Pour le 3ème des domaines logiques globaux, $DL_5^3(t)$, à l'union de l'union du 1er troisième domaine logique « normal » et du 3ème quatrième domaine logique «anormal», avec l'union du 2ème troisième domaine logique « peu normal » et du 2ème quatrième domaine logique « peu normal », et de l'union du 3ème troisième domaine logique « anormal » et du 1er quatrième domaine logique « normal » ;

d) Pour le 4ème des domaines logiques globaux, $DL_5^4(t)$, à l'union de l'union du 2ème troisième domaine logique « peu normal » et du 3ème quatrième domaine logique « anormal » et l'union du 3ème troisième domaine logique « anormal », et du 2ème quatrième domaine logique « peu normal » ;

e) Pour le 5ème des domaines logiques globaux, $DL_5^5(t)$, à l'union du 3ème troisième domaine logique « anormal » et du 3ème quatrième domaine logique « anormal » ;

Au cours d'une treizième étape **S113,** on attribue des probabilités d'appartenance globales ( $P(DL_5^I(t))$, avec I=1 à 5) de l'ensemble constitué par les premières dérivées des paramètres et les secondes dérivées à chacun des domaines logiques globaux ( $DL_5^I(t)$, avec I=1 à 5), de préférence en calculant lesdites probabilités comme suit :

$$P\left(DL_5^1(t)\right)= P\left(DL_3^1(t)\right)\times P\left(DL_4^1(t)\right)$$

$$P\left(DL_5^2(t)\right)= \left[P\left(DL_3^2(t)\right)\times P\left(DL_4^1(t)\right)\right]+\left[P\left(DL_3^1(t)\right)\times P\left(DL_4^2(t)\right)\right]$$

$$P\left(DL_5^3(t)\right)= \left[P\left(DL_3^3(t)\right)\times P\left(DL_4^1(t)\right)\right]+\left[P\left(DL_3^2(t)\right)\times P\left(DL_4^2(t)\right)\right]+\left[P\left(DL_3^1(t)\right)\times P\left(DL_4^3(t)\right)\right]$$

$$P\left(DL_5^4(t)\right)= \left[P\left(DL_3^3(t)\right)\times P\left(DL_4^2(t)\right)\right]+\left[P\left(DL_3^2(t)\right)\times P\left(DL_4^3(t)\right)\right]$$

$$P\left(DL_5^5(t)\right)= P\left(DL_3^3(t)\right)\times P\left(DL_4^3(t)\right)$$

Au cours d'une quatorzième étape **S114,** on norme le résultat obtenu en divisant chaque probabilité d'appartenance par la somme des probabilités d'appartenance aux 5 domaines logiques globaux.

Puis, au cours d'une quinzième étape **S115,** on qualifie la variabilité de la composition de l'effluent à partir des probabilités d'appartenance globales en utilisant, de préférence, uniquement le 5ème domaine logique global. On effectue les tests suivants (donnés à titres illustratifs):

- Si la probabilité d'appartenance au 5ème domaine logique global est supérieure à un premier seuil, par exemple 66%, alors cela signifie que la composition de l'effluent a fortement varié. Un feu rouge est affiché par l'interface **20.**
- Si la probabilité d'appartenance au 5ème domaine logique global est comprise par exemple entre 33% et 66% alors cela signifie que la composition de l'effluent a varié. Un feu orange est affiché par l'interface **20.**
- Si la probabilité d'appartenance au 5ème domaine logique global est inférieure à un second seuil, par exemple, 33% alors cela signifie que la composition de l'effluent n'a pas varié. Un feu vert est affiché par l'interface **20.**

Grâce à ce mode de mise en oeuvre, non limitatif, les fausses alertes sont avantageusement minimisées. En effet, il faut qu'une majorité des premières et deuxièmes dérivées présente des probabilités élevées d'appartenance aux domaines logiques « anormaux » pour que le 5ème domaine logique global présente une probabilité d'appartenance forte et déclenche une alarme.

Comme on l'a mentionné ci-dessus, les étapes **S107** à **S111** ne sont pas indispensables à la mise en oeuvre de l'invention.

En effet, une variante particulière conforme à l'invention, consiste à définir les domaines logiques globaux directement à partir des premiers et deuxièmes domaines logiques sans passer par des troisièmes et quatrièmes domaines logiques, à attribuer des probabilités d'appartenance globales de l'ensemble constitué par les premières dérivées des paramètres et les secondes dérivées à chacun des domaines logiques globaux, puis à qualifier la variabilité de la composition de l'effluent à partir des probabilités globales de manière similaire à ce qui est effectué au cours des étapes **S112** à **S115.**

## Revendications

**1.** Procédé de qualification de la variabilité de la composition d'un effluent, dans lequel on réalise une succession de mesures au cours du temps d'au moins un premier et un deuxième paramètres physico-chimiques de cet effluent; on détermine à chaque pas de temps une première dérivée pour chacun des premier et deuxième paramètres, ledit procédé étant **caractérisé en ce que**, à chaque pas de temps :

- on détermine au moins une deuxième dérivée entre les premier et deuxième paramètres ;
- on définit, pour la première dérivée de chacun des paramètres, des premiers domaines logiques comprenant au moins un premier domaine logique normal correspondant à une variabilité normale de ladite première dérivée, et un premier domaine logique anormal correspondant à une variabilité anormale de ladite première dérivée ;
- on attribue des premières probabilités d'appartenance à chacun des premiers domaines logiques pour la première dérivée de chacun des paramètres ;
- on définit des deuxièmes domaines logiques comprenant au moins un deuxième domaine logique normal correspondant à une variabilité normale de ladite deuxième dérivée, et un deuxième domaine logique anormal correspondant à une variabilité anormale de ladite deuxième dérivée ;
- on attribue des deuxièmes probabilités d'appartenance de la deuxième dérivée à chacun des deuxièmes domaines logiques ;
- on définit des domaines logiques globaux à partir des premiers et deuxièmes domaines logiques, les domaines logiques globaux comprenant au moins un domaine logique global normal et un domaine logique global anormal ;
- on attribue des probabilités d'appartenance globales de l'ensemble constitué par les premières dérivées des paramètres et au moins une deuxième dérivée à chacun des domaines logiques globaux ;
- on qualifie la variabilité de la composition de l'effluent à partir de ces probabilités d'appartenance globales.

2. Procédé de qualification selon la revendication 1, dans lequel, pour la première dérivée de chacun des paramètres, les premiers domaines logiques sont définis à partir d'un multiple de l'écart type évalué à partir d'un ensemble constitué par des valeurs de la première dérivée dudit paramètre déterminées à des pas de temps précédents.

3. Procédé de qualification selon la revendication 1 ou 2, dans lequel, pour la première dérivée de chacun des paramètres, les premiers domaines logiques sont également définis à partir de la moyenne ou de la médiane d'un ensemble constitué par des valeurs de la première dérivée dudit paramètre déterminées à des pas de temps précédents.

4. Procédé de qualification selon l'une quelconque des revendications 1 à 3, dans lequel, les deuxièmes domaines logiques sont définis à partir d'un multiple de l'écart type évalué à partir d'un ensemble constitué par des valeurs de ladite au moins une deuxième dérivée déterminées à des pas de temps précédents.

5. Procédé de qualification selon l'une quelconque des revendications 1 à 4, dans lequel, les domaines logiques globaux sont définis à partir d'un multiple de l'écart type évalué à partir d'un ensemble constitué par des valeurs des premières dérivées des paramètres et des valeurs de ladite au moins une deuxième dérivée déterminées à des pas de temps précédents.

6. Procédé de qualification selon l'une quelconque des revendications 1 à 5, dans lequel on définit en outre, pour la première dérivée de chacun des paramètres, un premier domaine logique intermédiaire correspondant à une variabilité peu normale de ladite première dérivée.

7. Procédé de qualification selon l'une quelconque des revendications 1 à 6, dans lequel on définit en outre, pour ladite au moins une deuxième dérivée, un deuxième domaine logique intermédiaire correspondant à une variabilité peu normale de ladite deuxième dérivée.

8. Procédé de qualification selon l'une quelconque des revendications 1 à 7, dans lequel on définit en outre un domaine logique global intermédiaire.

9. Procédé de qualification selon l'une quelconque des revendications 1 à 8, dans lequel les probabilités d'appartenance globales sont calculées à partir d'une somme pondérée des premières et deuxièmes probabilités d'appartenance.

10. Procédé de qualification selon l'une quelconque des revendications 1 à 8, dans lequel on définit en outre :

- des troisièmes domaines logiques comprenant au moins un troisième domaine logique normal et un troisième domaine logique anormal, les troisièmes domaines logiques étant définis à partir des premiers domaines logiques ;
- des quatrièmes domaines logiques comprenant au moins un quatrième domaine logique normal et un quatrième domaine logique anormal, les quatrièmes domaines logiques étant définis à partir des deuxièmes domaines logiques ;

procédé dans lequel :

- on attribue en outre des troisièmes probabilités d'appartenance d'un ensemble constitué par les premières dérivées des paramètres à chacun des troisièmes domaines logiques ;
- on attribue en outre des quatrièmes probabilités d'appartenance d'un ensemble constitué par au moins ladite au moins une deuxième dérivée à chacun des quatrièmes domaines logiques ;

procédé dans lequel :

- les domaines logiques globaux sont définis à partir des troisièmes et quatrièmes domaines logiques.

11. Procédé de qualification selon la revendication 10, dans lequel les probabilités d'appartenance globales sont calculées à partir d'une somme pondérée des troisièmes et quatrièmes probabilités d'appartenance.

12. Procédé de qualification selon la revendication 11, dans lequel ladite somme pondérée fait intervenir au moins un coefficient de pondération dynamique.

13. Procédé de qualification selon l'une quelconque des revendications 1 à 12, dans lequel le domaine logique global qualifiant la variabilité de la composition de l'effluent est celui pour lequel la probabilité d'appartenance globale est maximale.

14. Procédé de qualification selon l'une quelconque des revendications 1 à 12, dans lequel le domaine logique global qualifiant la variabilité de la composition de l'effluent est le domaine logique le plus anormal pour lequel la probabilité d'appartenance globale est non nulle.

15. Procédé de qualification selon l'une quelconque des revendications 1 à 12, dans lequel le domaine logique global qualifiant la variabilité de la composition de l'effluent est le domaine logique le plus anormal pour lequel la probabilité d'appartenance globale est supérieure à un seuil prédéterminé.

16. Procédé de qualification selon l'une quelconque des revendications 1 à 15, dans lequel la première dérivée est une dérivée temporelle.

17. Procédé de qualification selon l'une quelconque des revendications 1 à 16, dans lequel le premier paramètre est l'absorbance de l'effluent mesuré pour une première longueur d'onde.

18. Procédé de qualification selon l'une quelconque des revendications 1 à 17, dans lequel chacun des paramètres est l'absorbance de l'effluent pour une longueur d'onde donnée.

19. Procédé de qualification selon la revendication 18, dans lequel la première dérivée est une dérivée de l'absorbance par rapport au temps pour une longueur d'onde donnée, tandis que la deuxième dérivée est une dérivée de l'absorbance par rapport à la longueur d'onde calculée à un pas de temps donné.

20. Dispositif (10) de suivi de la variabilité de la composition d'un effluent mettant en oeuvre le procédé selon l'une quelconque des revendications 1 à 19 et comportant des moyens de mesure (12), des moyens de mémoire (16) et un microprocesseur (18).


**Claims**

1. A method of qualifying the variability of the composition of an effluent, in which a succession of measurements is effected over time of at least one first physical-chemical parameter and one second physical-chemical parameter of the effluent; and
determining at each time step a first derivative for each of the first and second parameters, said method being **characterized by** the following actions at each time step:

- determining at least one second derivative between the first and second parameters;
- defining first logical domains for the first derivative of each of the parameters comprising at least one normal first logical domain corresponding to a normal variability of said first derivative and one abnormal first logical domain corresponding to an abnormal variability of said first derivative;
- assigning first probabilities of the first derivative of each of the parameters belonging to each of the first logical domains;
- defining second logical domains comprising at least one normal second logical domain corresponding to a normal variability of said second derivative and one abnormal second logical domain corresponding to an abnormal variability of said second derivative;
- assigning second probabilities of the second derivative belonging to each of the second logical domains;
- defining global logical domains on the basis of the first and second logical domains, the global logical domains comprising at least one normal global logical domain and one abnormal global logical domain;
- assigning global probabilities of the combination of the first derivatives of the parameters and at least one second derivative belonging to each of the global logical domains; and
- qualifying the variability of the composition of the effluent on the basis of said global probabilities.

2. A qualification method according to claim 1, wherein, for the first derivative of each of the parameters, the first logical domains are defined on the basis of a multiple of the standard deviation evaluated from a set consisting of values of the first derivative of said parameter determined in preceding time steps.

3. A qualification method according to claim 1 or claim 2, wherein, for the first derivative of each of the parameters, the first logical domains are also defined on the basis of the mean or median value of a set consisting of values of the first derivative of said parameter determined in preceding time steps.

4. A qualification method according to any one of claims 1 to 3, wherein the second logical domains are defined on the basis of a multiple of the standard deviation evaluated on the basis of a set consisting of values of said at least one second derivative determined in preceding time steps.

5. A qualification method according to any one of claims 1 to 4, wherein the global logical domains are defined on the basis of a multiple of the standard deviation evaluated on the basis of a set consisting of the values of the first derivatives of the parameters and values of said at least one second derivative determined in preceding time steps.

6. A qualification method according to any one of claims 1 to 5, wherein, for the first derivative of each of the parameters, a first intermediate logical domain is defined corresponding to a subnormal variability of said first derivative.

7. A qualification method according to any one of claims 1 to 6, wherein, for said at least one second derivative, a second intermediate logical domain is defined corresponding to a subnormal variability of said second derivative.

8. A qualification method according to any one of claims 1 to 7, wherein an intermediate global logical domain is further defined.

9. A qualification method according to any one of claims 1 to 8, wherein said global probabilities are calculated on the basis of a weighted sum of said first and second probabilities.

10. A qualification method according to any one of claims 1 to 8, wherein:

- third logical domains comprising at least a normal third logical domain and an abnormal third logical domain are defined, the third logical domains being defined on the basis of the first logical domains;
- fourth logical domains comprising at least a normal fourth logical domain and an abnormal fourth logical domain are defined, the fourth logical domains being defined on the basis of the second logical domains;
- third probabilities of a set consisting of the first derivatives of the parameters belonging to each of the third logical domains are assigned;
- fourth probabilities of a set consisting at least of said at least one second derivative belonging to each of the fourth logical domains are assigned;
- the global logical domains are defined on the basis of the third and fourth logical domains.

11. A qualification method according to claim 10, wherein said global probabilities are calculated on the basis of a weighted sum of said third and fourth probabilities.

12. A qualification method according to claim 11, wherein said weighted sum involves at least one dynamic weighting coefficient.

13. A qualification method according to any one of claims 1 to 12, wherein the global logical domain qualifying the variability of the composition of the effluent is that having the maximum global probability.

14. A qualification method according to any one of claims 1 to 12, wherein the global logical domain qualifying the variability of the composition of the effluent is the most abnormal logical domain for which said global probability is non-zero.

15. A qualification method according to any one of claims 1 to 12, wherein the global logical domain qualifying the variability of the composition of the effluent is the most abnormal logical domain for which said global probability is above a predetermined threshold.

16. A qualification method according to any one of claims 1 to 15, wherein the first derivative is a time derivative.

17. A qualification method according to any one of claims 1 to 16, wherein the first parameter is the absorbance of the effluent measured at a first wavelength.

18. A qualification method according to any one of claims 1 to 17, wherein each of the parameters is the absorbance of the effluent at a given wavelength.

19. A qualification method according to claim 18, wherein the first derivative is a derivative of the absorbance relative to time for a given wavelength and the second derivative is a derivative of the absorbance relative to wavelength calculated in a given time step.

20. A device (10) for monitoring the variability of the composition of an effluent using the method according to any one of claims 1 to 19 and including measuring means (12), memory means (16), and a microprocessor (18).

**Patentansprüche**

1. Verfahren zur Bewertung der Variabilität der Zusammensetzung eines Abwassers, bei dem im Laufe der Zeit eine Folge von Messungen wenigstens eines ersten und eines zweiten physikalisch-chemischen Parameters dieses Abwassers durchgeführt wird, zu jedem Zeitschritt eine erste Ableitung für jeden der ersten und zweiten Parameter bestimmt wird, wobei das Verfahren **dadurch gekennzeichnet ist, daß** zu jedem Zeitschritt:

- wenigstens eine zweite Ableitung zwischen den ersten und zweiten Parametern bestimmt wird,
- für die erste Ableitung eines jeden der Parameter erste logische Bereiche definiert werden, die wenigstens einen ersten normalen logischen Bereich, der einer normalen Variabilität der ersten Ableitung entspricht, sowie einen ersten anormalen logischen Bereich, der einer anormalen Variabilität der ersten Ableitung entspricht, umfassen,
- jedem der ersten logischen Bereiche für die erste Ableitung eines jeden der Parameter erste Zugehörigkeitswahrscheinlichkeiten zugeordnet werden,
- zweite logische Bereiche definiert werden, die wenigstens einen zweiten normalen logischen Bereich, der einer normalen Variabilität der zweiten Ableitung entspricht, sowie einen zweiten anormalen logischen Bereich, der einer anormalen Variabilität der zweiten Ableitung entspricht, umfassen,
- zweite Zugehörigkeitswahrscheinlichkeiten der zweiten Ableitung jedem der zweiten logischen Bereiche zugeordnet werden,
- anhand der ersten und zweiten logischen Bereiche logische Gesamtbereiche definiert werden, wobei die logischen Gesamtbereiche wenigstens einen normalen logischen Gesamtbereich und einen anormalen logischen Gesamtbereich umfassen,
- jedem der logischen Gesamtbereiche Gesamtzugehörigkeitswahrscheinlichkeiten der von den ersten Ableitungen der Parameter und von wenigstens einer zweiten Ableitung gebildeten Einheit zugeordnet werden,
- anhand dieser Gesamtzugehörigkeitswahrscheinlichkeiten die Variabilität der Zusammensetzung des Abwassers bewertet wird.

2. Bewertungsverfahren nach Anspruch 1, wobei für die erste Ableitung eines jeden der Parameter die ersten logischen Bereiche mit Hilfe eines Vielfachen der Standardabweichung definiert werden, die anhand einer Einheit berechnet wird, welche von zu vorhergehenden Zeitschritten bestimmten Werten der ersten Ableitung des Parameters gebildet ist.

3. Bewertungsverfahren nach Anspruch 1 oder 2, wobei für die erste Ableitung eines jeden der Parameter die ersten logischen Bereiche auch mit Hilfe des Mittelwertes oder des Medianwertes einer Einheit definiert werden, die von zu vorhergehenden Zeitschritten bestimmten Werten der ersten Ableitung des Parameters gebildet ist.

4. Bewertungsverfahren nach einem der Ansprüche 1 bis 3, wobei die zweiten logischen Bereiche mit Hilfe eines Vielfachen der Standardabweichung definiert werden, die anhand einer Einheit berechnet wird, welche von zu vorhergehenden Zeitschritten bestimmten Werten der wenigstens einen zweiten Ableitung gebildet ist.

5. Bewertungsverfahren nach einem der Ansprüche 1 bis 4, wobei die logischen Gesamtbereiche mit Hilfe eines Vielfachen der Standardabweichung definiert werden, die anhand einer Einheit berechnet wird, welche von Werten der ersten Ableitungen der Parameter und von Werten der wenigstens einen zweiten Ableitung, die zu vorhergehenden Zeitschritten bestimmt werden, gebildet ist.

6. Bewertungsverfahren nach einem der Ansprüche 1 bis 5, wobei ferner für die erste Ableitung eines jeden der Parameter ein erster logischer Zwischenbereich definiert wird, der einer anormalen Variabilität der ersten Ableitung entspricht.

7. Bewertungsverfahren nach einem der Ansprüche 1 bis 6, wobei ferner für die wenigstens eine zweite Ableitung ein zweiter logischer Zwischenbereich definiert wird, der einer anormalen Variabilität der zweiten Ableitung entspricht.

8. Bewertungsverfahren nach einem der Ansprüche 1 bis 7, wobei ferner ein logischer Gesamtzwischenbereich definiert wird.

9. Bewertungsverfahren nach einem der Ansprüche 1 bis 8, wobei die Gesamtzugehörigkeitswahrscheinlichkeiten mit Hilfe einer gewichteten Summe aus den ersten und zweiten Zugehörigkeitswahrscheinlichkeiten berechnet werden.

10. Bewertungsverfahren nach einem der Ansprüche 1 bis 8, wobei ferner folgendes definiert wird:

   - dritte logische Bereiche, die wenigstens einen dritten normalen logischen Bereich und einen dritten anormalen logischen Bereich umfassen, wobei die dritten logischen Bereiche anhand der ersten logischen Bereiche definiert werden,
   - vierte logische Bereiche, die wenigstens einen vierten normalen logischen Bereich und einen vierten anormalen logischen Bereich umfassen, wobei die vierten logischen Bereiche anhand der zweiten logischen Bereiche definiert werden,
   Verfahren bei dem:

   - ferner dritte Zugehörigkeitswahrscheinlichkeiten einer Einheit bestehend aus den ersten Ableitungen der Parameter einem jeden der dritten logischen Bereiche zugeordnet werden,
   - ferner vierte Zugehörigkeitswahrscheinlichkeiten einer Einheit bestehend aus wenigstens der wenigstens einen zweiten Ableitung einem jeden der vierten logischen Bereiche zugeordnet werden,

   Verfahren, bei dem:

   - die logischen Gesamtbereiche anhand der dritten und vierten logischen Bereiche definiert werden.

11. Bewertungsverfahren nach Anspruch 10, wobei die Gesamtzugehörigkeitswahrscheinlichkeiten mit Hilfe einer gewichteten Summe aus den dritten und vierten Zugehörigkeitswahrscheinlichkeiten berechnet werden.

12. Bewertungsverfahren nach Anspruch 11, wobei die gewichtete Summe wenigstens einen dynamischen Gewichtungskoeffizienten einbezieht.

13. Bewertungsverfahren nach einem der Ansprüche 1 bis 12, wobei der logische Gesamtbereich, der die Variabilität der Zusammensetzung des Abwassers bewertet, derjenige ist, bei dem die Gesamtzugehörigkeitswahrscheinlichkeit maximal ist.

14. Bewertungsverfahren nach einem der Ansprüche 1 bis 12, wobei der logische Gesamtbereich, der die Variabilität der Zusammensetzung des Abwassers bewertet, der anormalste logische Bereich ist, bei dem die Gesamtzugehörigkeitswahrscheinlichkeit ungleich null ist.

15. Bewertungsverfahren nach einem der Ansprüche 1 bis 12, wobei der logische Gesamtbereich, der die Variabilität der Zusammensetzung des Abwassers bewertet, der anormalste logische Bereich ist, bei dem die Gesamtzugehörigkeitswahrscheinlichkeit oberhalb einer vorbestimmten Schwelle liegt.

**16.** Bewertungsverfahren nach einem der Ansprüche 1 bis 15, wobei die erste Ableitung eine zeitliche Ableitung ist.

**17.** Bewertungsverfahren nach einem der Ansprüche 1 bis 16, wobei der erste Parameter die für eine erste Wellenlänge gemessene Absorbanz des Abwassers ist.

**18.** Bewertungsverfahren nach einem der Ansprüche 1 bis 17, wobei jeder der Parameter die Absorbanz des Abwassers für eine gegebene Wellenlänge ist.

**19.** Bewertungsverfahren nach Anspruch 18, wobei die erste Ableitung eine Ableitung der Absorbanz bezogen auf die Zeit für eine gegebene Wellenlänge ist, während die zweite Ableitung eine Ableitung der Absorbanz bezogen auf die Wellenlänge, zu einem gegebenen Zeitschritt berechnet, ist.

**20.** Vorrichtung (10) zum Verfolgen der Variabilität der Zusammensetzung eines Abwassers, die das Verfahren nach einem der Ansprüche 1 bis 19 zur Anwendung bringt und Meßmittel (12), Speichermittel (16) sowie einen Mikroprozessor (18) umfaßt.

**FIG.1**

**FIG.2**

**FIG.3**

**FIG.4**

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2783322
- FR 2787883

- EP 1070954 A